(19) Europäisches Patentamt
European Patent Office
Office européen des brevets

(11) Publication number: **0 070 084**
**A2**

# (12) EUROPEAN PATENT APPLICATION

(21) Application number: 82302004.5

(22) Date of filing: 20.04.82

(51) Int. Cl.$^3$: C 07 D 233/50
A 61 K 31/415

(30) Priority: 24.04.81 GB 8112692

(43) Date of publication of application: 19.01.83 Bulletin 83/3

(84) Designated Contracting States: BE CH DE FR GB IT LI NL SE

(71) Applicant: BEECHAM GROUP PLC
Beecham House Great West Road
Brentford Middlesex(GB)

(72) Inventor: Baker, Geoffrey Harold
Flat 2 11 Brambledon Road
Wallington Surrey(GB)

(72) Inventor: Beeley, Lee James
40 St. Johns Raod Westcott
Dorking Surrey(GB)

(72) Inventor: Newsome, Peter Martin
41 St. Clair Drive
Worcester Park Surrey(GB)

(72) Inventor: Richards, David Hugh
'Calgary' Brentbrook Park
North Holmwood Dorking Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

## (54) Imidazoline derivatives.

(57) Compounds of formula (I):

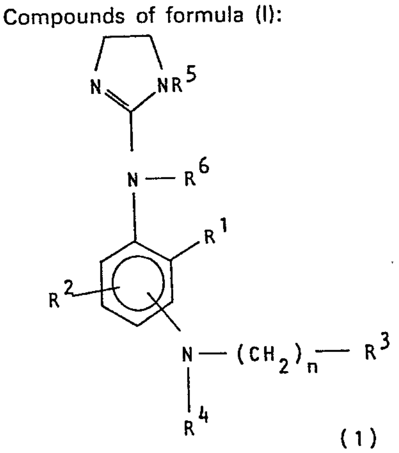

(1)

wherein
$R^1$ is halogen, alkyl or alkoxy;
$R^2$ is hydrogen, halogen, alkyl or alkoxy;
$R^3$ is an optionally substituted, saturated, unsaturated or aromatic, carbo- or hetero-, mono- or bi-cyclic group;
$R^4$ is hydrogen or alkyl,

$$-\underset{R^4}{\underset{|}{N}}-(CH_2)_n-R^3$$

is located meta or para to the imidazolinylamino group; one of $R^5$ and $R^6$ is hydrogen and the other is hydrogen, alkyl or acyl; and
N is 0, 1, 2 or 3,
or an acid addition salt thereof, have antidiarrhoeal activity.

EP 0 070 084 A2

# Imidazoline Derivatives

The present invention relates to a class of novel 2-phenylaminoimidazoline derivatives and their use in human and veterinary medicine.

European Patent Application No. 0 043 659 discloses compounds of formula

A - B - C

wherein A is 2-imidazolinyl optionally substituted on a nitrogen atom with $C_{1-4}$ alkyl.

B is oxygen, sulphur, $CR^1R^2$ wherein $R^1$ and $R^2$ are the same or different and each is selected from $C_{1-4}$ alkyl and hydrogen, or $NR^3$ wherein $R^3$ is selected from $C_{1-4}$ alkyl and hydrogen;

and C is a 5- or 6- membered carbo- or heterocyclic group having a nitrogen-containing substituent and optionally also substituted by one or two groups, each selected from halogen, $C_{1-4}$ alkyl, $C_{1-4}$ alkoxy and hydroxy; or an N-acyl derivative and/or an acid addition salt thereof.

as being useful in the treatment of diarrhoea in humans and non-human animals.

It has now been found that certain phenylamino imidazoline derivatives, having nitrogen-containing substituents on the phenyl group but which are not specifically disclosed in EP 0 043 659, are particularly suitable for use in treating diarrhoea in human and non-human animals.

Accordingly, the present invention provides a compound of formula (I)

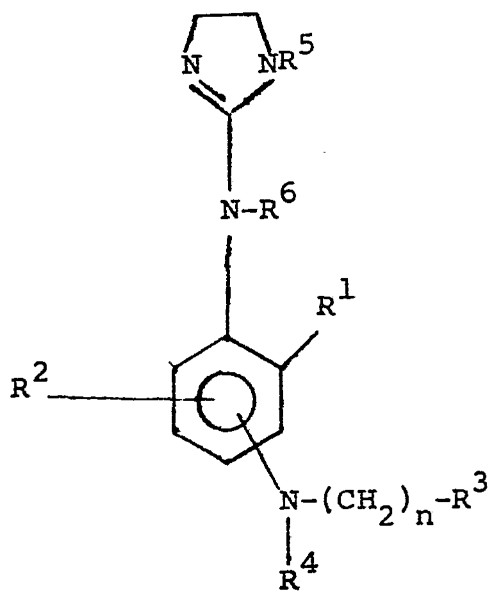

(I)

wherein $R^1$ is halogen, alkyl or alkoxy;

$R^2$ is hydrogen, halogen, alkyl or alkoxy;

$R^3$ is an optionally substituted saturated, unsaturated or aromatic, carbo- or hetero-, mono- or bicyclic group;

$R^4$ is hydrogen or alkyl;

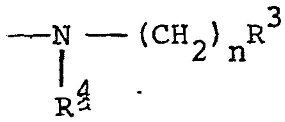 is located meta or para to the imidazolinylamino group;

one of $R^5$ and $R^6$ is hydrogen and the other is hydrogen, alkyl or acyl;

and n is 0, 1, 2 or 3

or an acid addition salt thereof.

Suitably $R^3$ is unsubstituted or substituted with halogen, alkyl, alkoxy, amino, mono- or di-alkylamino, mono- or di-acylamino, nitro, hydroxy, hydroxyalkyl, alkoxyalkyl, mercapto, mercaptoalkyl, alkylthio, amidino guanidino alkylthioalkyl, sulphonyl, sulphonamide or cyano or 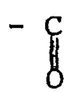 - X where X is OH or an ester or amide thereof.

Compounds of formula (I) wherein $R^6$ is hydrogen may exist in the tautomeric form (I)

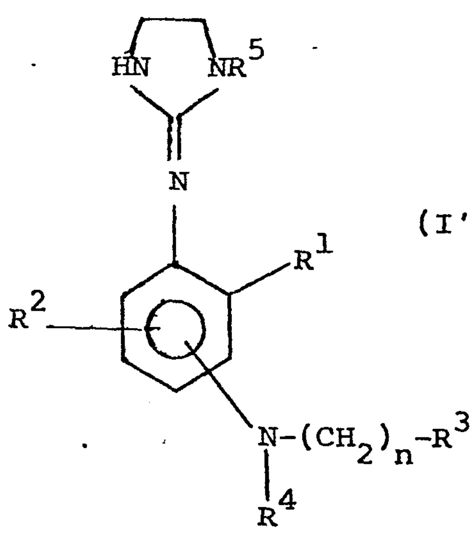

(I')

which is also encompassed by the present invention.

As used herein the term "alkyl" refers to straight, branched or cyclic lower alkyl groups, preferably having from 1-4 carbon atoms. Such alkyl groups may themselves be optionally substituted, for instance with halogen atoms.

As used herein the term "acyl" refers to the residues of carboxylic acids, such as lower alkylcarboxylic acids, and benzoic acid.

Particular examples of groups $R^3$ include phenyl, pyridyl naphthalenyl, quinolinyl, isoquinolinyl and indolyl groups

and partially or fully saturated analogues thereof.

Preferably $R^2$ is located ortho to the imidazolinylamino group.

Preferably $\underset{R^4}{\underset{|}{N}} - (CH_2)_nR^3$ is located para to the imidazolinylamino group.

A particularly preferred group of compounds within formula (I) are those having the formula (IA)

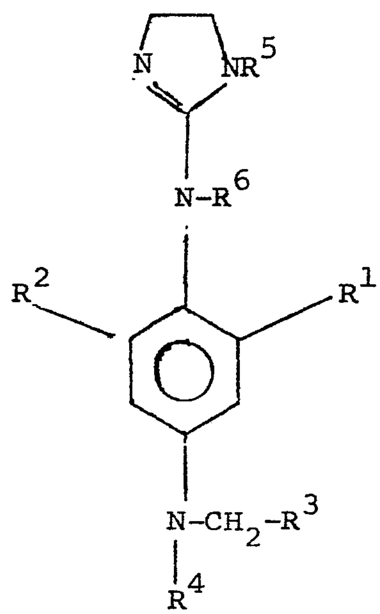

(IA)

wherein $R^1$, $R^2$, $R^3$, $R^4$, $R^5$ and $R^6$ are as defined above and acid addition salts thereof.

Suitable acid addition salts include salts of the following acids:-

hydrochloric, hydrobromic, hydroiodic, nitric, sulphuric, citric, tartaric and pamoic acids.

Compounds of formula (I) inhibit intestinal secretion and are therefore useful in the treatment of enterotoxin induced diarrhoea, such as in E.coli scours in livestock E.coli and related diarrhoeas in humans. Compounds of

formula (I) also have α-agonist activity and/or antihypertensive, vasocontricting and sedative activity.

According to the present invention there is also provided a process for producing a compound of formula (I) which process comprises:

(a) reacting a compound of formula (II)

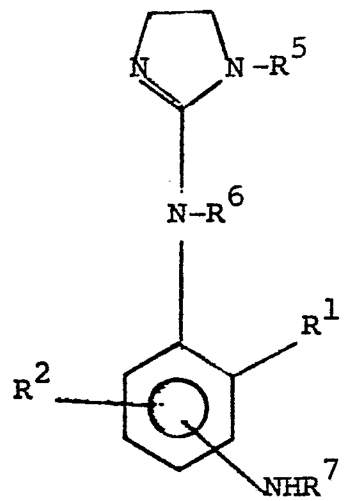

(II)

wherein $R^7$ is alkyl, $(CH_2)_nR^3$ or hydrogen with a compound of formula (III)

$$R^8 - X \qquad \text{(III)}$$

wherein $R^8$ is $(CH_2)_nR^3$ when $R^7$ is alkyl or hydrogen
or $R^8$ is alkyl when $R^7$ is $(CH_2)_nR^3$
and X is a reactive leaving group;

(b) reacting a compound of formula (IV)

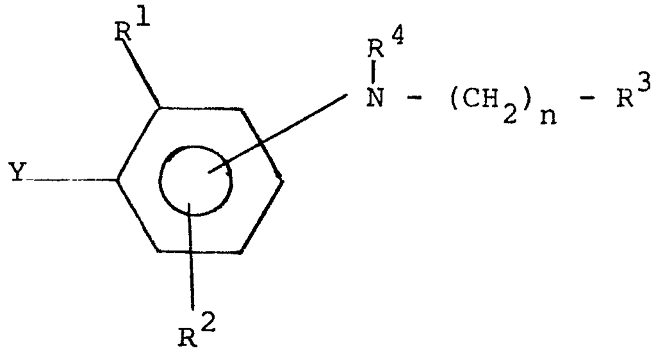

(IV)

wherein $R^1$, $R^2$ $R^3$, $R^4$ and n are as defined with respect to formula (I)

and Y is a group 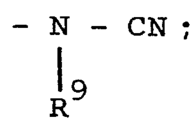 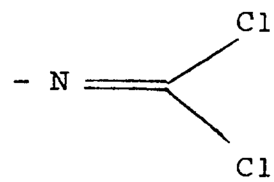 ;

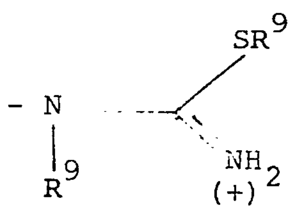 or 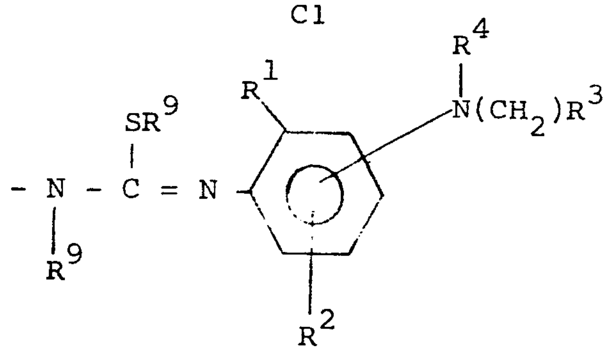

wherein each group $R^9$ is selected from hydrogen and $(C_{1-4})$ alkyl, with a compound of formula (V).

$$H_2N - CH_2 - CH_2 - NHR^{10} \qquad (V)$$

wherein $R^{10}$ is hydrogen or $(C_{1-4})$ alkyl; or

(c) reacting a compound of formula (VI)

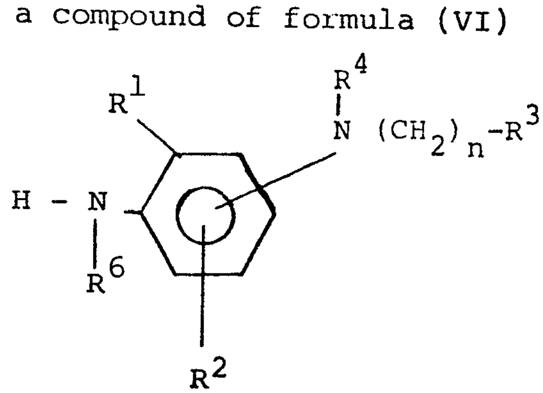

(VI)

wherein $R^1$, $R^2$, $R^3$ and $R^6$ are as defined with respect to formula (I) with a compound of formula (VII)

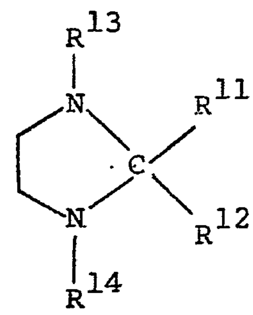

(VII)

wherein $R^{11}$ and $R^{12}$ together form an oxo group;
$R^{13}$ is hydrogen, $(C_{1-4})$ alkyl, or acyl;
and $R^{14}$ is hydrogen;
or $R^{12}$ and $R^{14}$ together form a bond and
$R^{11}$ is $(C_{1-4})$ alkylthio or benzylthio
and $R^{13}$ is hydrogen or $(C_{1-4})$ alkyl; or

(d) reacting a compound of formula (VIII)

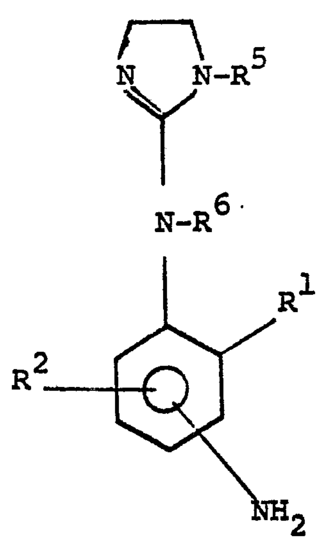

(VIII)

with a compound of formula (IX)

$$R^3 - (CH_2)_m - \underset{\underset{O}{\|}}{C} - Z \qquad (IX)$$

wherein m is 0,1 or 2

and z is hydrogen, halogen or $- \mathrm{O} - \underset{\underset{\mathrm{O}}{\|}}{\mathrm{C}} - R^{16}$

and $R^{16}$ is hydrogen, alkyl, or $-(CH_2)_m - R^3$

and thereafter reducing the product to the requisite compound of formula (I);

and optionally (e), forming a salt of the compound of formula (I).

In the above processes it may be advantageous to protect the nitrogen atoms not involved in the desired reaction. Such protection may be effected by conventional means and the protecting groups may be removed conventionally.

In process (a) X is conveniently a halogen atom, especially chlorine or bromine or an O-tosyl or O-mesyl group but other conventional leaving groups may be employed.

Process (b) may be conducted according to the process described in UK Patent No. 1,229,995 when

Y is - 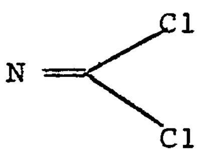 ;

according to the process described in Netherlands Patent No. 6,411,516 (Chem. Abs. 63 P18102 g)

when Y is - 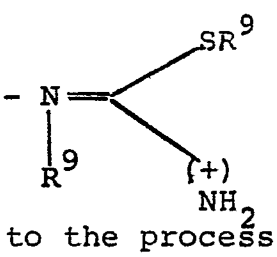 ;

according to the process described in Chem. Abs., 85, P94364b when

Y is 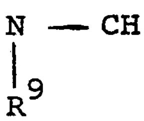 ,

and according to the process described in Chem. Abs., 85, P5633 g when

Y is - 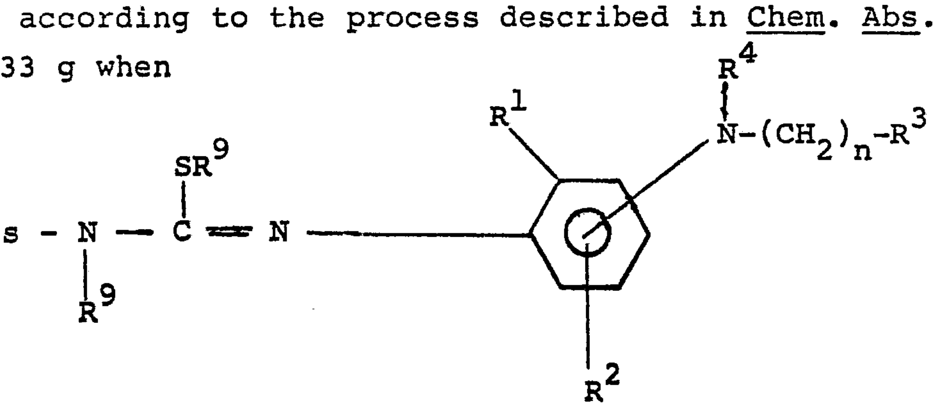

Process (c) may be conducted according to the process described in German Offenlegungschrift 2,505,297 or Netherlands Patent No. 7,404,472 when $R^{11}$ and $R^{12}$ together form an oxo group, and according to the process described in Chem. Abs., 84, P59465t when $R^{14}$ and $R^{12}$ together form a bond.

Process (d) may be conducted for instance by reacting the compound of formula (VIII) with an aldelyde and reducing the product with a complex hydride reducing agent such as cyanoborohydride. Alternatively the process can be effected by acylation of the amino group followed by conventional reduction using, for instance, diborane-dimethylsulphide complex or using lithium aluminium hydride. Such process as described in :-

B Rouot and G LeClerc, Bull.Soc.Chim.Fr., 520, (1979)
D Atlas and S L Sabol, Biochem. and Biohys. Res. Comm., 94, 924, (1980)
C V Wilson and J F Stenberg, Org. Syn. Coll. Vol.4, 564, (1963)

Compounds of formula (II) may be produced by processes analogous to those described above.

Alternatively compounds of formula (II) may be produced by reducing the corresponding compound having a nitro substituent on the phenyl group to provide the required amino substituent.

Compounds of formula (IIA)

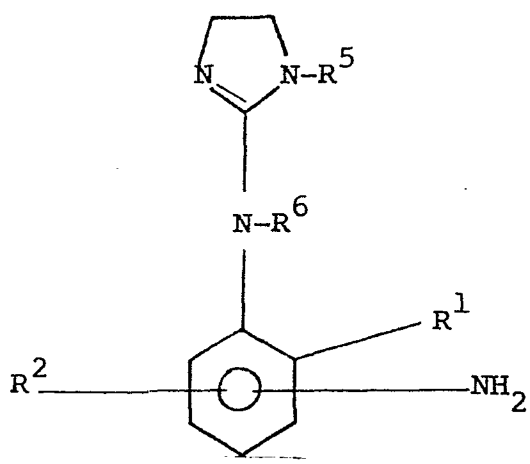

(IIA)

wherein $R^1$, $R^2$, $R^5$ and $R^6$ are as defined with respect to formula (I) and the amino group is at one of the meta positions with respect to the imidazolinylamino group, are novel and form a further aspect of the present invention. Such compounds are useful as intermediates

in the production of compounds of formula (I). They also have a sedative activity in their own right and are accordingly useful in human and veterinary medicine.

Compounds of formula (IIA) are generically disclosed in European Patent Application No. 0 035 393 as having anovulatory activity. However that Patent Application, whilst mentioning aminophenyliminoimidazolines does not give any indication of which position on the phenyl ring is occupied by the amino substituent. Accordingly there is no specific disclosure of the compounds of formula (IIA) and these are, therefore, novel.

Compounds of formula (IIA) may be produced by processes described above under (b) and (c) in relation to the production of compounds of formula (I) but using appropriate starting materials of formulae (IV) or (VI) wherein the group $NR^4(CH_2)_nR^3$ is replaced by $NH_2$, or a protected derivative thereof or by reducing the corresponding compound having a nitro-group in place of the amino group by conventional chemical or catalytic processes.

Compounds of formula (IIA) and their acid addition salts may be produced according to the processes described generally with respect to production of compounds of formula (II) above.

The starting materials described above may be produced by standard methods as may acid addition salts of compounds of formulae (I) and (IIA).

The present invention further provides a compound of formula (I) or a compound of formula (IIA) for use in human or veterinary medicine.

The present invention further provides a pharmaceutical composition comprising a compound of formula (I) or a compound of formula (IIA) and a pharmaceutically acceptable carrier therefor.

As used herein the term "pharmaceutical composition" includes compositions suitable for human and/or animal use and "pharmaceutically acceptable" includes veterinarily acceptable.

Pharmaceutical compositions of compounds of formulae (I) and (IIA) will, of course, be adapted for administration to the humans or animals to be treated. Thus, for example, the composition may be a shaped composition, such as a bolus, tablet or capsule. In such cases the pharmaceutically acceptable carrier will be chosen from the usual range of lubricants, dispersants, binders, fillers and the like. When these shaped compositions are for administration to cattle and pigs often they will weigh at least 1 g, on occasions at least 2 g.

For administration to humans, especially children, the drug may suitably be presented as a syrup including suitable colouring and/or flavouring agents. Such syrups are conveniently presented in unit or multi-dose containers.

For veterinary use the composition may also be a dispersion or a solution of a compound of formula (I) or formula (IIA), (hereinafter referred to as 'the drug') in a suitable vehicle for use with an oral doser (this is a well known item of farm equipment, basically comprising a liquid reservoir, a mouthpiece adapted for insertion into animal mouths, and a pump mechanism whereby unit doses can be ejected from the reservoir through the mouthpiece). Conveniently the drug may be administered from an oral doser as an aqueous solution. Alternatively the vehicle will be an oil or water based cream to ensure homogeneity of the unit doses administered.

The invention, therefore, also provides an oral doser containing a multi-dose of the drug in a veterinarily acceptable vehicle.

The drugs of the invention may also be added to the animal feed or drinking water. Thus the invention also provides animal feed or animal drinking water containing a compound of formula (I) or (IIA). It will be convenient to formulate these animal feed and drinking water compositions with a multi-dose of the drug so that the animal takes in an appropriate quantity of the drug along with its diet. It will also be convenient to present the composition of the invention as pre-mixes for addition to the feed or drinking water.

With human babies or young animals, a particularly useful technique is to blend their milk with the drugs of this invention.

The compositions of the invention may also be formulated for injection. In such cases the drug chosen is suitably dissolved in water for injection.

Often it will be appropriate to include in the compositions a further medicine such as an antibacterial agent for example an antibiotic such as amoxycillin or neomycin or a sulphonamide such as sulfadoxin.

Treatment of diarrhoea and scours using a compound of formula (I) may be supplemented by oral rehydration therapy such as that described in U.K. Patent No. 1 581 826, German Offenlegungsschrift No. 28 54 281 and US Pätent No. 4 164 568. Conveniently the compound of formula (I) may be administered with the oral rehydration formulation. Alternatively it may be provided separately and administered simultaneously or sequentially with the oral rehydration formulation.

Accordingly the present invention provides, in a particular aspect, a formulation for treating diarrhoea which comprises an effective non-toxic amount of a compound of formula (I) as hereinbefore defined and an oral rehydration composition comprising a pharmacologically acceptable aqueous solution containing at least 0.5% w/v of an actively absorbed monosaccharide, at least 25 mM sodium ions and having an osmolarity less than 500 m Osmolar.

Preferably the oral rehydration composition further comprises actively-absorbed amino acids and electrolytes.

Active absorption (or active transport) is well known to the skilled man, as are the monosaccharides and amino acids which are actively absorbed. In this regard the reader is referred to standard text books such as 'Medicinal Physiology' by Guyton (published by W.B. Saunders and Company) 4th Edition pages 769 to 771. Of course whether or not a particular monosaccharide or amino acid is actively absorbed may also readily be determined by experiment as for example described in Wilson T.H. 1962 Intestinal Absorption (Saunders, Philadelphia).

- - -

To be actively absorbed, monsaccharides must have (a) at least six carbon atoms in their chain (b) a D-pyranose ring structure and (c) an intact hydroxyl group at carbon 2. Thus suitable examples of monosaccharides for use in this invention include the naturally occurring D-pyranoses such as glucose and galactose. Other examples of suitable monosaccharides include naturally occurring D-pyranoses that have been chemically modified whilst retaining the necessary structural features (a), (b) and (c). Examples of such modified monosaccharides include $C_{2-7}$ acylated and $C_{1-4}$ alkylated derivatives, such as acetyl, methyl, ethyl and n- and iso-propyl derivatives. Specific examples include α-methyl glucoside, 3-O-methyl glucose and 6-deoxygalactose.

Preferably the monosaccharide will be glucose or galactose. The monosaccharide of choice for use in this invention is glucose (e.g. dextrose).

Suitably the actively absorbed monosaccharide will account for 40 to 80% of the composition, more suitably 50 to 75% for example 60 to 75% of the composition. Often the monosaccharide will represent at least 65% of the composition.

Suitable examples of actively absorbed naturally occurring amino acids include neutral amino acids such as glycine and alanine and basic amino acids such as arginine. Preferably the amino acid is glycine.

Suitably the amino acid will account for 7.5 to 30% of the composition, preferably 7.5 to 20%, for example 8 to 15% and especially 8 to 12% of the composition.

Suitable electolytes for such inclusion include salts containing ions such as sodium, potassium, calcium, magnesium, chloride, phosphate, gluconate, sulphate, bicarbonate, carbonate and the like. Other favoured electrolytes for inclusion in the compositions include potassium dihydrogen phosphate, dipotassium hydrogen phosphate, tripotassium phosphate, potassium chloride and the like, with potassium dihydrogen phosphate being particularly suitable.

One particularly preferred electolyte for inclusion in the composition of the invention is sodium chloride.

Suitably the oral rehydration formulation comprises 10 to 25% electrolytes. Preferably, when sodium chloride is used as electrolyte, it will account for from 7 to 20% of the formulation, for example 10 to 16% of the formulation.

The oral rehydration composition may optionally contain citric acid and/or a salt thereof, representing from 0.5 to 10% of the composition. Suitably 0.5 to 5%, preferably 0.5 to 2%, for example 0.6 to 1.2% of the composition. Often the composition will contain both citric acid and a salt thereof, but the combined amount will not exceed 10% of the composition. Suitable salts of citric acid include sodium and potassium salts such as mono-, di, or tri-sodium or mono-, di- or tri-potassium citrate. Often the composition will include 0.1 to 5% of a salt of citric acid, more suitably 0.1 to 0.5% of such a salt.

The improved oral rehydration formulation may be presented as a dry powder for dissolution in water or a concentrate for dilution in water. Alternatively the formulation may be an orally administrable solution in water, in which case the solution contains at least 0.5% w/v of the monosaccharide and at least 25 mM sodium ions and has an osmolarity of less than 500 m Osmolar. Preferably the formulation also comprises an actively absorbed amino acid and electrolytes.

Most preferably the formulation comprises a compound of formula (I) and an oral rehydration formulation as described in U.K. Patent No. 1 581 826 and U.K. Patent Application No. 2 012 163 A, U.S. Patent No. 3 898 328 or Nalin, D.R. & Cash, R.A., Bull World Health Org, 43, 361, (1970) and French Patent No. 2 467 599, which disclosures are incorporated herein by reference.

Advantageously the improved formulation includes a preservative or antioxidant such as ascorbate anions or sodium metabisulphite. When presented in solution the formulation would preferably comprise about 6mM ascorbate or up to 0.1% w/v sodium metabisulphite.

Advantageously the formulation, when presented as a dry powder, is packaged to exclude air and moisture.

Preferably such compositions contain glucose as the monosaccharide and glycine as the amino acid.

Thus a particularly preferred composition of the invention comprises a compound of formula (I), 60 to 75% glucose, 8 to 15% glycine, 0.5 to 2% citric acid, 0.1 to 0.5% of a salt of citric acid, and 10 to 16% sodium chloride. Such compositions often include 5 to 10% of potassium dihydrogen phosphate.

A second suitable veterinary composition comprises 40 to 80% of an actively absorbed monosaccharide, 7.5 to 13% of an actively absorbed naturally occurring amino acid, and 0.5 to 10% of a citrate salt.

The citrate salt represents 0.5 to 10% of this composition. More suitably the salt will represent 0.5 to 5%, preferably 0.5 to 2%, for example 0.6 to 1.2% of the composition. Suitable examples of citrate salts include sodium or potassium salts such as mono-, di- or tri-sodium, or mono-, di- or tri-potassium citrate.

Thus a particularly preferred composition of the invention comprises a compound of formula (I), 60 to 75% glucose, 8 to 12% glycine, 0.5 to 2% of a salt of citric acid, and 10 to 16% sodium chloride. Such compositions often include 5 to 10% of potassium dihydrogen phosphate.

These compositions suitably contain at least 65% monosaccharide.

If desired the compositions of this invention can contain other substances such as vitamins, minerals, buffers, excipients or the like in conventional manner.

In general the compositions of this invention will be in the form of dry powder for example one which is readily soluble in water. However in an alternative aspect the compositions of this invention will comprise an aqueous solution containing dissolved therein the previously defined solutes in the previously defined relative proportions.

The powders of this invention may be prepared by mixing together the individual components in conventional manner. Once mixed the composition may be put into sachets or other conventional containers.

It is frequently advantageous to separate the monosaccharide component from the other components of the composition. This can be effected by using double sachets or other double containers. In such cases components other than the monosaccharide can be mixed and filled into one half of the double sachet and the monosaccharide can be filled into the other half of the double sachet. In such form the compositions of the invention have been found to be particularly stable. The compound of formula (I) may be included in either sachet.

The composition of the invention will normally be administered to the diarrhoeic animal in the form of an aqueous solution, by the oral route. Such solutions may for example contain 20 to 45g./litre of the composition, suitably 25 to 35b./litre, for example 30g./litre. In general calves will be administered from 2 to at least 4 litres per day of such solutions while piglets will normally be administered from a quarter to a one litre per day. The solutions may be administered ad libitum or in two to four or more equal doses per day or by any other similar conventional regime.

It will be realised that in the treatment of severely scouring animals anti-bacterial agents may be administered in conjunction with the compositions of the invention. Examples of suitable anti-bacterial agents for such use include ampicillin, amoxycillin and tetracyclines.

The skilled man will realise that the effective absorption properties found with the liquid compositions of the invention will enable them to be used with advantage whenever liquid absorption by animals is a problem. For example the compositions may be used in treating the general dehydration found in post-operative conditions in animals such as dogs and cats. They may also be administered with advantage to stressed animals, such as recently purchased calves and the like. It is however believed that the compositions of the invention will be of the greatest use in the treatment of diarrhoea in calves.

The amount of drug administered must, of course, be sufficient to bring about the desired effect. This will depend on the effect desired, (for instance, different dosages may be required for treatment of diarrhoea and for sedation) as well as on the body weight of the recipient and the chosen route of administration. Thus, by way of example, useful dosage units of the composition for treating diarrhoea may contain 1 μg to 50 mg of the drug, more suitably 20 μg to 20 mg. Of course, it will be appreciated that many preferred compositions of the invention are in multi-dose form as, for the therapy of animals, it is often most desirable to be able rapidly to treat a number of animals. Such multi-dose compositions will contain, by way of example, at least 1 mg of the drug. Depending on the exact nature of the said multi-dose composition, often it will contain at least 50 mg of the drug, and on occasions as much as 1 g. Doses may be administered once or several times daily.

The present invention further provides a method for treating humans and animals, which method comprises adminstering an effective, non-toxic amount of a compound of formula (I) or of formula (IIA) to the sufferer. Such treatment may be sedation, vasoconstriction, or a treatment for diarrhoea or hypertension.

In a particular aspect the method of treatment comprises the administration of a pharmaceutical composition of a compound of formula (I) or of formula (IIA), as hereinbefore described.

The present invention will now be illustrated by the following Examples which are not intended to limit the invention in any way.

Example 1

2-(2-Chloro-5-nitrophenylimino) imidazolidine

2-chloro-5-nitroaniline (17.26g, 100mM) was dissolved in phosphoryl chloride (135ml). 1-Acetyl-2-imidazolidone (14g, 110mM) was added and the reaction mixture stirred at 50°C for 48 hours. The solvent was concentrated under reduced pressure and cold water carefully added. The solution was washed with dichloromethane before neutralization with ammonia. The resultant precipitate was filtered to give 16g of product.

This product was hydrolysed with dilute sodium hydroxide in methanol to give 2-(2-chloro-5-nitrophenylimino) imidazolidine in quantitive yield m.pt = 198-200°C.

Example 2

2-(5-Amino-2-chlorophenylimino) imidazolidine, hydrochloride

A solution of concentrated hydrochloric acid (12ml) in aqueous ethanol (50%, 110 ml)was added dropwise to a stirred heated mixture of 2-(2-chloro-5-nitrophenylimino) imidazolidine (33g, 137mM) and iron powder (38.5g, 86mM) in aqueous ethanol (50%, 310 ml). The mixture was refluxed for 2 hours after which time it was filtered hot and evaporated at reduced pressure to give a dark oil. Trituration with hot acetonitrile caused precipitation and 2-(5-amino-2-chlorophenylimino) imidazolidine, hydrochloride was filtered off. Yield = 25.0g, m.pt = 183-186$^{\circ}$C. An analytical sample was crystallized from acetonitrile.

Analysis calculated for $C_9H_{12}Cl_2N_4$

reqd. C : 43.74 ; H : 4.89; N : 22.66; Cl : 28.70
found. C : 43.70 ; H : 4.84; N : 22.66; Cl : 28.80

Example 3

2-(3-amino-2,6-dichlorophenylimino) imidazolidine

To a solution of 2-(3-nitro-2,6-dichlorophenylimino) imidazolidine (1.9 g, 3.2 x $10^{-3}$ mole) in concentrated hydrochloric acid (4 ml, 4.0 x $10^{-2}$ mole) and ethanol (4 ml) was slowly added stannous chloride (2.0 g, 8.8 x $10^{-3}$ mole) with stirring. The mixture was stirred (1 hour) at room temperature before addition of sodium hydroxide solution (2M) to adjust to pH 10.0 . The inorganic precipitate was filtered off and the filtrate evaporated to give a white solid. This solid was triturated with a solution of dichloromethane (10 mls) and ethanol (10 mls) which was then evaporated to give the required 2-(3-amino-2, 6-dichlorophenylimino) imidazolidine (0.2 g 25% yield). m.pt = 171-173$^{o}$C).

Analysis calculated for $C_9H_{10}Cl_2N_4$

| | | | | |
|---|---|---|---|---|
| Theoretical : | C 44.11 ; | H 4.11 ; | N 22.85 ; | Cl 28.93 ; |
| Found : | C 43.49 ; | H 4.26 ; | N 22.86 ; | Cl 28.84 ; |

Example 4

2-(4-Benzylamino-2,6-dichlorophenylimino)imidazolidine hydrochloride

To a solution of 2-(4-amino-2,6-dichlorophenylimino)imidazolidine (2.4 g, 8.5 x $10^{-3}$ mole) in ethanol (150 ml) and water (100 ml) was added benzaldehyde (0.9 g, 8.5 x $10^{-3}$ mole) then sodium cyanoborohydride (1.5 g, 2.55 x $10^{-2}$ mole) slowly over 3 hours. The mixture was stirred at room temperature (18 hours). Excess sodium cyanoborohydride was destroyed by adjusting the pH of the solution to 1.0 with concentrated hydrochloric acid and the mixture stirred (2 hours) before evaporation of the solvent at reduced pressure gave the crude product. Crystallization from hot water (50 ml) afforded 2-(2,6-dichloro-4-benzyl amino phenyl imino) imidazolidine, hydrochloride (1.9 g, 55% yield)

Analysis calculated for $C_{16}H_{17}Cl_3N_4$

| | | | | |
|---|---|---|---|---|
| reqd. | C : 51.70 ; | H : 4.61 ; | N : 15.07 ; | Cl : 28.62 |
| found | C : 51.90 ; | H : 5.08 ; | N : 14.75 ; | Cl : 28.58 |

A sample was purified for analysis by dissolving 1.0 g of compound in hot water (50 ml) and slowly adding an excess of saturated sodium iodide solution. The resulting crystalline precipitate was filtered off as the hydroiodide salt in quantitative yield (m.pt = 250-252°C)

Analysis calculated for $C_{16}H_{17}Cl_2N_4I$

Theorètical : C 41.50 ; H 3.70 ; N 12.09 ; Cl 15.31 ; I 27.40

Found : C 41.42 ; H 3.79 ; N 11.94 ; Cl 15.41 ; I 27.60

Example 5

2-[4-(4-Chlorobenzylamino)-2,6-dimethylphenylimino] imidazolidine, hydrochloride

A mixture of 2-(4-amino-2,6-dimethylphenylimino) imidazolidine (2.4g, 10mM), 4-chlorobenzaldehyde (1.46g, 10mM) 3A$^{o}$ molecular sieves (2.4g), platinum oxide (50mg) and ethanol (100ml) was hydrogenated for 48 hours at low pressure. The solution was filtered before evaporation at reduced pressure to give an oil. The product was dissolved in dichloromethane and washed with water. The organic layer was dried (sodium sulphate), filtered and evaporated at reduced pressure to give 2-[4-(4-chlorobenzylamino)-2,6-dimethylphenylimino] imidazolidine, hydrochloride (2.0g). Recrystallization from acetonitrile gave an analytical sample m.pt = 204-206$^{o}$C .

Analysis calculated for $C_{18}H_{22}Cl_2N_4$:

reqd. C : 59.19 ; H : 6.08 ; N : 15.32 ; Cl : 19.41
found C : 59.13 ; H : 5.80 ; N : 15.33 ; Cl : 19.43

Example 6

2-[2,6-Dichloro-4-(2-thenylamino) phenylimino] imidazolidine, hydrochloride

2-Thiophene carboxaldehyde (0.95g, 8.5mM) and 2-(4-amino-2,6-dichlorophenylimino) imidazolidine (2.4g, 8.5mM) were reacted as described in Example 4 to give 2-[2,6-dichloro-4-(2-thenylamino) phenylimino] imidazolidine, hydrochloride (0.55g) m.pt = 205-208$^{O}$C.

Analysis calculated for $C_{14}H_{15}Cl_3N_4S$.

reqd. C : 44.53 ; H : 4.00 ; N : 14.83 ; Cl : 28.17 ; S : 8.47
found C : 44.22 ; H : 4.13 ; N : 14.68 ; Cl : 28.32 ; S : 7.99

Example 7

2-[2,6-Dichloro-4-(3,4-methylenedioxybenzylamino) phenylimino] imidazolidine, hydrochloride

Piperonal (1.28 g, 8.5mM) and 2-(4-amino-2,6-dichlorophenylimino) imidazolidine (2.4g, 8.5mM) were reacted as described in Example 4 to give 2-[2,6-dichloro-4-(3,4-methylenedioxybenzylamino) phenylimino] imidazolidine (1.4g) m.pt = 207-209°C.

Analysis calculated for $C_{17}H_{17}Cl_3N_4O_2$

| | | | | |
|---|---|---|---|---|
| reqd. | C : 49.11 ; | H : 4.12 ; | N : 13.47 ; | Cl : 25.59 |
| found | C : 49.07 ; | H : 4.24 ; | N : 13.48 ; | Cl : 25.22 |

Example 8

2-[4-(4-Chlorobenzylamino)-2,6-dichlorophenylimino] imidazolidine, hydroiodide

4-Chlorobenzaldehyde (0.6g, 4.5 mM) and 2-(4-amino-2,6-dichlorophenylimino)imidazolidine (1.2g, 4.25 mM) were reacted as described in Example 4 and a sample converted to the hydroiodide salt to give 2-[4-(4-chlorobenzylamino)-2,6-dichlorophenylimino] imidazolidine, hydroiodide (100 mg) m.pt = 222-225$^{o}$C.

Analysis calculated for $C_{16}H_{16}Cl_3N_4I$

reqd. C : 38.62 ; H : 3.24 ; N : 11.25 ; Cl : 21.38 ; I : 25.50
found C : 37.93 ; H : 3.14 ; N : 11.32 ; Cl : 21.36 ; I : 25.51

## Example 9

### 2-[4-(4-Carboxybenzylamino)-2,6-dichlorophenylimino] imidazolidine, hydrochloride

4-carboxybenzaldehyde (1.28g, 8.5mM) and 2-(4-amino-2,6-dichlorophenylimino) imidazolidine (1.2g, 4.25mM) were reacted as described in Example 4 to give 2-[4-(4-carboxybenzylamino)-2,6-dichlorophenylimino] imidazolidine, hydrochloride (1.0g) m.pt = 202-204°C.

Analysis calculated for $C_{17}H_{17}Cl_3N_4O_2$

| | | | | |
|---|---|---|---|---|
| reqd. | C : 49.12 ; | H : 4.12 ; | N : 13.47 ; | Cl : 25.59 ; |
| found | C : 49.43 ; | H : 4.35 ; | N : 13.06 ; | Cl : 25.49 ; |

Example 10

2-[4-(4-Carbomethoxybenzylamino)-2,6-dichlorophenylimino] imidazolidine, hydrochloride

The compound of Example 9 was dissolved in methanol and gaseous hydrogen chloride bubbled through the solution. After heating under reflux for 2 hours the solvent was evaporated at reduced pressure to give 2-[4-(4-carbomethoxybenzylamino)-2,6-dichlorophenylimino] imidazolidine, hydrochloride (2.0g) which was purified by chromatography using silica gel HF and 5% methanol/dichloromethane as eluant. Yield = 0.8g m.pt = 235-240°C.

Analysis calculated for $C_{18}H_{19}Cl_3N_4O_2$

| | | | | |
|---|---|---|---|---|
| reqd. | C : 50.31 | H : 4.40 | N : 13.03 | Cl : 24.76 |
| found | C : 50.46 | H : 4.46 | N : 13.05 | Cl : 24.89 |

Example 11

2-[2,6-Dichloro-4-(4-fluorobenzylamino) phenylimino] imidazolidine, hydrochloride

4-Fluorobenzaldehyde (0.53g, 4.25 mM) and 2-(4-amino-2,6-dichlorophenylimino) imidazolidine (1.2g, 4.25 mM) were reacted as described in Example 4 to give 2-[2,6-dichloro-4-(4-fluorobenzylamino) phenylimino] imidazolidine, hydrochloride (0.2g) m.pt = 270°C

Analysis calculated for $C_{16}H_{16}Cl_3N_4F$

reqd. C : 49.32 ; H : 4.14 ; N : 14.37

found C : 49.42 ; H : 4.12 ; N : 14.33

Example 12

2-[4-(5-Bromo-2-thenylamino)-2,6-dichlorophenylimino] imidazolidine, hydrochloride

5-Bromothiophene-2-carboxaldehyde (0.8g, 4.25 mM) and 2-(4-amino-2,6-dichlorophenylimino) imidazolidine (1.2g, 4.25mM) were reacted as described in Example 4 to give 2-[4-(5-bromo-2-thenylamino)-2,6-dichlorophenylimino] imidazolidine, hydrochloride, (1.0g) m.pt = 229-231$^{o}$C

Analysis calculated for $C_{14}H_{14}BrCl_3NS$

| | | | | |
|---|---|---|---|---|
| reqd. | C : 36.83 ; | H : 3.09 ; | N : 12.27 ; | S : 7.00 |
| found | C : 36.80 ; | H : 3.09 ; | N : 11.70 ; | S : 6.63 |

Example 13

2- [2,6-Dichloro-4-(4-methylbenzylamino) phenylimino] imidazolidine, hydrochloride

4-Tolualdehyde (1.2g, 4.25 mM) and 2-(4-amino-2,6-dichlorophenylimino) imidazolidine (1.2g, 4.25 mM) were reacted as described in Example 4 to give 2- [2,6-dichloro-4-(4-methylbenzylamino) phenylimino] imidazolidine, hydrochloride (0.33g) m.pt = 252-254°C.

Analysis calculated for $C_{17}H_{18}Cl_3N_4$

reqd. C : 53.07 ; H : 4.72 ; N : 14.55 ; Cl : 27.65
found C : 52.94 ; H : 5.06 ; N : 14.21 ; Cl : 28.08

Example 14

2-[4-(3-Chlorobenzylamino)-2,6-dichlorophenylimino] imidazolidine, hydrochloride

3-Chlorobenzaldehyde (1.2g, 8.5 mM) and 2-(4-amino-2, 6-dichlorophenylimino)imidazolidine (2.4g, 8.5 mM) were reacted as described in Example 4 to give 2-[4-(3-chlorobenzylamino)-2,6-dichlorophenylimino] imidazolidine, hydrochloride (2.0g) m.pt = 210-212$^{o}$C

Analysis calculated for $C_{16}H_{16}Cl_{4}N_{4}$

reqd. C : 47.31 ; H : 3.97 ; N : 13.79 ; Cl : 34.92
found C : 46.95 ; H : 4.05 ; N : 13.57 ; Cl : 35.19

Example 15

2-[2,6-Dichloro-4-(4-methylthiobenzylamino) phenylimino] imidazolidine, hydrochloride

4-(methylthio) benzaldehyde (1.3g, 8.5 mM) and 2-(4-amino-2,6-dichlorophenylimino)imidazolidine (2.4g, 8.5 mM) were reacted as described in Example 4 to give 2-[2,6-dichloro-4-(4-methylthiobenzylamino) phenylimino] imidazolidine, hydrochloride (1.3g) m.pt = 155-157$^{o}$C.

Analysis calculated for $C_{17}H_{19}Cl_3N_4S$

| | | | | | |
|---|---|---|---|---|---|
| reqd. | C : 48.88 ; | H : 4.59 ; | N : 13.41 ; | Cl : 25.46 ; | S : 7.66 |
| found | C : 48.59 ; | H : 4.59 ; | N : 12.96 ; | Cl : 25.63 ; | S : 7.41 |

Example 16

2-[4-(4-Bromobenzylamino)-2,6-dichlorophenylimino] imidazolidine, hydrochloride

4-Bromobenzaldehyde (1.6g, 8.5 mM) and 2-(4-amino-2, 6-dichlorophenylimino)imidazolidine (2.4g, 8.5 mM) were reacted as described in Example 4 to give 2-[4-(4-bromobenzylamino)-2,6-dichlorophenylimino] imidazolidine, hydrochloride (1.0g) m.pt = 163-165°C.

Analysis calculated for $C_{16}H_{16}BrCl_3N_4$

reqd. C : 42.65 ; H : 3.58 ; N : 12.43 ; $Cl^-$: 7.87
found C : 42.32 ; H : 3.78 ; N : 11.96 ; $Cl^-$: 7.56

Example 17

2-[2,6-Dichloro-4-(3-methoxybenzylamino phenylimino] imidazolidine, hydrochloride

3-Anisaldehyde (1.2g, 8.5 mM) and 2-(4-amino-2,6-dichlorophenylamino)imidazolidine (2.4, 8.5 mM) were reacted as described in Example 3 to give 2-[2,6-dichloro-4-(3-methoxybenzylamino) phenylimino] imidazoldine, hydrochloride (1.0g) m.pt = 210-215°C dec.

Analysis calculated for $C_{17}H_{19}Cl_3N_4O$

reqd. C : 50.82 ; H : 4.77 ; N : 13.94 ; Cl : 26.48
found C : 51.23 ; H : 4.80 ; N : 13.78 ; Cl : 26.01

Example 18

2-[2,6-Dichloro-4-(4-isopropyl benzylamino) phenylimino] imidazolidine

4-Isopropylbenzaldehyde (1.2g, 8.5 mM) and 2-(4-amino-2,6-dichlorophenylimino)imidazolidine (2.4g, 8.5 mM) were reacted as described in Example 4 to give (2.0g) crude product. Purification of the neutralized product using chromatography (alumina and 5% methanol/dichloromethane as eluant), afforded 2-[2,6-dichloro-4-(4-isopropyl benzylamino) phenylimino]imidazolidine (1.0g) m.pt 205-207°C.

Analysis calculated for $C_{19}H_{22}Cl_2N_4$

reqd. C : 60.48 ; H : 5.88 ; N : 14.84 ; Cl : 18.80
found C : 59.97 ; H : 5.79 ; N : 14.52 ; Cl : 19.22

Example 19

2-(4-Benzylamino-2,6-dimethylphenylimino)imidazolidine hydrochloride

Benzaldehyde (4.25g, 40 mM) and 2-(4-amino-2,6-dimethylphenylimino)imidazolidine (9.6g, 40 mM) were reacted as described in Example 4, gave crude 2-(4-benzylamino-2,6-dimethylphenylimino)imidazolidine, hydrochloride (13.0g). Chromatography using silica gel HF eluting with 5% methanol dichloromethane afforded an analytical sample m.pt 106-107$^{O}$C.

Analysis calculated for $C_{18}H_{23}ClN_4$

reqd. C : 65.35 ; H : 7.01 ; 16.93 ; Cl : 10.72

found C : 65.06 ; H : 7.16 ; 16.90 ; Cl : 10.50

Mass spectrum observed m/e = 294.1833 $M^+$

Calculated for $C_{18}H_{22}N_4$ = 294.1844

Example 20

2-[2,6-Dimethyl-4-(3-methoxybenzylamino) phenylimino] imidazolidine, hydrochloride

A mixture of 3-anisaldehyde (1.4g, 10 mM), 2-(4-amino-2,6-dimethylphenylimino)imidazolidine (4.8g, 20 mM) and molecular sieves (4.8g) in ethanol (100 ml) were heated under reflux for 12 hours. After cooling to room temperature, platinum oxide (50 mg) was added and the mixture hydrogenated until no further uptake of hydrogen was observed. The solution was filtered through diatomaceous earth and the solvent evaporated at reduced pressure to give an oil.

This oil was dissolved in dichloromethane and washed with water. The organic layer was dried (sodium sulphate), filtered and an excess of ethanolic hydrochloric acid added. Evaporation of the solvents gave an oily solid. 2-[2,6-dimethyl-4-(3-methoxybenzylamino) phenylimino] imidazolidine, hydrochloride (0.5g) was obtained by the addition of hot acetonitrile and filtering off the resultant precipitate. m.pt = 173-175°C.

Analysis calculated for $C_{19}H_{26}Cl_2N_4O$

reqd. C : 57.42 ; H : 6.60 ; N : 14.09 ; Cl : 17.84
found C : 56.94 ; H : 6.45 ; N : 14.03 ; Cl : 17.21

Example 21

2-[4-(4-Carbomethoxybenzylamino)-2,6-dimethylphenylimino] imidazolidine, hydrochloride

4-Carboxybenzaldehyde (1.5g, 10 mM) and 2-(4-amino-2, 6-dimethylphenylimino)imidazolidine (2.4g, 10 mM) were reacted as described in Example 20 to afford 2-[4-(4-carboxybenzyl)-2,6-dimethylphenylimino]imidazolidine, hydrochloride 3.0g. The methyl ester was then prepared by the method described in Example 10. Purification by chromatography using silica gel HF and 5% methanol/dichloromethane as eluant followed by recrystallization from acetonitrile give 2-[4-(4-carbomethoxybenzylamino)-2,6-dimethylphenylimino]imidazolidine, hydrochloride (1.0g) m.pt = 215-216°C.

Mass spectrum, observed m/e = 352.1897 $M^+$

Calculated for $C_{20}H_{24}N_4O_2$ = 352.1897

Example 22

2-(5-Benzylamino-2-chlorophenylimino)imidazolidine

Benzaldehyde (1.0g, 9.4 mM) and 2-(5-amino-2-chlorophenylimino) imidazolidine hydrochloride (1.0g, 4.7 mM) were reacted as described in Example 5. The solution was filtered and evaporated at reduced pressure to give an oil which was recrystallized from water to give 0.9g of crude product. The product was purified by chromatography using alumina and 2% ethanol/chloroform as eluant, affording 2-(5-benzylamino-2-chlorophenylimino)imidazolidine (0.4g). Recrystallization from acetonitrile gave a sample, m.pt = 165-166°C.

Analysis calculated for $C_{16}H_{17}ClN_4$

reqd. C : 63.88 ; H : 5.70 ; N : 18.62 ; Cl : 11.78
found C : 63.84 ; H : 5.70 ; N : 18.67 ; Cl : 11.86

Example 23

2-[5-(4-Bromobenzylamino)-2-chlorophenylimino]imidazolidine, hydrochloride

4-Bromobenzaldehyde (0.85g, 4.7 mM) and 2-(5-amino-2-chlorophenylimino)imidazolidine hydrochloride (1.0g, 4.7 mM) were reacted as described in Example 22. The product was purified by recrystallization from water (20ml) to yield 2-[5-(4-bromobenzylamino)-2-chlorophenylamino] imidazolidine hydrochloride (0.2g) m.pt = 105-106°C.

Mass spectrum, observed m/e = 378.023 ($M^+$)

Calculated for $C_{16}H_{16}BrClN_4$ = 378.0245

Example 24

2-[2,6-Dichloro-4-(4-hydroxymethylbenzylamino) phenylimino] imidazolidine,

2-[4-(4-Carbomethoxybenzylamino)-2,6-dichlorophenylimino] imidazolidine hydrochloride (1.0g, 2.3 mM) was stirred in dry tetrahydrofuran (60 ml) under a nitrogen atmosphere. Lithium Aluminium Hydride (200mg, 5.2 mM) was slowly added over a period of 30 minutes. The mixture was heated to 50°C and stirring continued for 2 hours. Excess lithium aluminium hydride was destroyed by addition of ethylacetate (3.0ml), ethanol (3.0ml) then water (3.0ml). The solvents were removed by evaporation at reduced pressure to give an oily solid.

The oily solid was extracted with chloroform (100ml) which after drying(sodium sulphate)and filtering was evaporated at reduced pressure to give 0.8g of crude product. Recrystallization from acetonitrile (25ml) afforded 2-[5-(4-bromobenzylamino)-2-chlorophenylimino] imidazolidine, (0.4g) m.pt = 203-205°C.

Mass spectrum, observed m/e = 364.0842

Calculated for $C_{17}H_{18}Cl_2N_4O$ = 364.0855

Biological Evaluation of the Compounds -

(i) Anti-Scour activity

The following test was carried out in mice:

7-9 day old infant mice are separated from their mothers shortly before use and are administered the compound 45 mins prior to oral challenge with 0.05-0.10 ml of culture filtrate containing the enterotoxin ST prepared from an enteropathogenic strain of *E. coli*. Control animals receive drug vehicle 45 mins prior to challenge with a similar amount of culture filtrate. The compounds are administered orally. Animals are killed two hours later and the entire intestine removed. The ratio of gut weight to remaining bodyweight (GW/BW) is determined from each animal and the increase in this ratio is determined by substracting 0.06 (GW/BW for untreated mice) from the GW/BW of the animal. Drug treated animals are compared with untreated controls. If the compound has had an effect in inhibiting the fluid secretion caused by the enterotoxin(s) present in the culture filtrate then the gut weight/bodyweight ratio should be reduced in the treated animals. The percentage fluid inhibition is determined from the formula:

$$100 - \left[\frac{\text{Mean increase in GW/BW ratio in treated animals}}{\text{Mean increase in GW/BW ratio in control animals}}\right] \times 100$$

Results are given in table 1

ACTIVITY OF COMPOUNDS IN ST CHALLENGED MICE - TABLE 1 0070084

| Compound Described in Example No. | Salt | Dose level mg/Kg p.o. | % Inhibition | Significance (Students t) |
|---|---|---|---|---|
| 4 | HI | 200 | 67 | P < 0.01 |
| | | 50 | 66 | P < 0.001 |
| | | 10 | 62 | P < 0.001 |
| | | 1 | 63 | P < 0.01 |
| | | 0.1 | 46 | P < 0.05 |
| | | 0.01 | 9 | |
| 4 | HCl | 10 | 52 | P < 0.001 |
| | | 2 | 49 | P < 0.001 |
| | | 0.4 | 35 | P < 0.001 |
| 5 | HCl | 250 | 100 | P < 0.001 |
| | | 50 | 46 | P < 0.01 |
| | | 10 | 21 | |
| 5 | HI | 50 | 44 | P < 0.05 |
| 6 | HCl | 200 | 96 | P < 0.001 |
| | | 50 | 85 | P < 0.001 |
| | | 10 | 44 | P < 0.001 |
| | | 1 | 30 | P < 0.05 |
| | | 0.1 | 2 | |
| 7 | HCl | 200 | 59 | P < 0.001 |
| | | 50 | 55 | P < 0.001 |
| | | 10 | 24 | |
| | | 1 | 21 | |
| | | 0.1 | 23 | P < 0.05 |
| | | 0.01 | 9 | |

| Compound Described in Example No. | Salt | Dose level mg/Kg p.o. | % Inhibition | Significance (Students t) |
|---|---|---|---|---|
| 8 | HI | 50 | 44 | P < 0.05 |
| | | 10 | 18 | |
| 9 | HCl | 250 | 30 | P < 0.001 |
| | | 50 | 26 | P < 0.05 |
| | | 10 | 8 | |
| 10 | HCl | 250 | 55 | P < 0.001 |
| | | 50 | 47 | P < 0.001 |
| | | 10 | 30 | P < 0.01 |
| | | 2 | 20 | P < 0.05 |
| 11 | HCl | 200 | 71 | P < 0.001 |
| | | 50 | 47 | P < 0.001 |
| | | 10 | 48 | P < 0.001 |
| | | 1 | 31 | |
| 12 | HCl | 200 | 69 | P < 0.001 |
| | | 50 | 61 | P < 0.001 |
| | | 10 | 61 | P < 0.001 |
| | | 1 | 49 | P < 0.001 |
| | | 0.1 | 51 | P < 0.001 |
| 13 | HCl | 200 | 40 | P < 0.01 |
| | | 50 | 22 | |
| | | 10 | 24 | |
| | | 1 | 28 | |
| | | 0.1 | 12 | |

| Compound Described in Example No. | Salt | Dose level mg/Kg p.o. | % Inhibition | Significance (Students t) |
|---|---|---|---|---|
| 14 | HCl | 200 | 73 | P < 0.01 |
| | | 50 | 48 | P < 0.01 |
| | | 10 | 64 | P < 0.001 |
| | | 1 | 54 | P < 0.001 |
| | | 0.1 | 43 | P < 0.01 |
| 15 | HCl | 200 | 38 | P < 0.05 |
| | | 50 | 54 | P < 0.01 |
| | | 10 | 56 | P < 0.01 |
| 16 | HCl | 200 | 83 | P < 0.001 |
| | | 50 | 74 | P < 0.001 |
| | | 10 | 63 | P < 0.001 |
| | | 1 | 27 | |
| 17 | HCl | 200 | 85 | P < 0.001 |
| | | 50 | 21 | P < 0.001 |
| | | 10 | 21 | P < 0.001 |
| | | 1 | 37 | P < 0.01 |
| 18 | | 250 | 51 | P < 0.01 |
| | | 50 | 43 | P < 0.01 |
| | | 10 | 61 | P < 0.01 |

| Compound Described in Example No. | Salt | Dose level mg/Kg p.o. | % Inhibition | Significance (Students t) |
|---|---|---|---|---|
| 19 | HCl | 50 | 66 | $P < 0.01$ |
| | | 10 | 32 | $P < 0.05$ |
| | | 2 | 19 | |
| | | 0.4 | 1 | |
| 20 | 2HCl | 50 | 58 | $P < 0.001$ |
| | | 10 | 50 | $P < 0.01$ |
| | | 2 | 36 | |
| | | 0.4 | 38 | $P < 0.05$ |
| 21 | HCl | 250 | 46 | $P < 0.001$ |
| | | 50 | 54 | $P < 0.01$ |
| | | 10 | 17 | |
| 22 | | 250 | 20 | |
| 24 | | 250 | 47 | $P < 0.01$ |
| | | 50 | 28 | $P < 0.05$ |
| | | 10 | 18 | |

(ii) Antihypertension activity

Compounds were made up in 0.9% w/v sterile saline and were administered as doses of 10 µg free base in 10 µl to anaesthetised rats by injection into the lateral cerebral venticles (I.C.V.) as described by Hayden et al., Life Sci., 5, 1509-15, (1966). Changes in blood pressure and heart rate were recorded over a period of 1 hour via a cannula inserted in the carotid artery.

Results are shown in Table 2.

Table 2

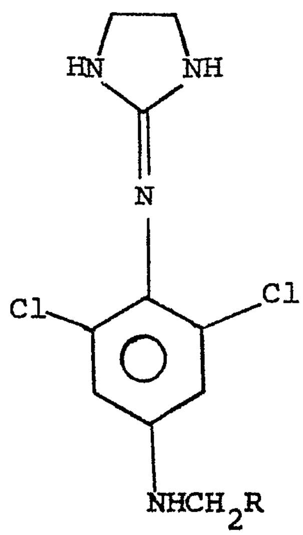

| Compd Number | R | N | Diastolic Blood Pressure | | Heart Rate | |
|---|---|---|---|---|---|---|
| | | | Pre-dose (mmHg) | Max % Change | Pre-dose (beats/ min) | Max % change |
| 1 | 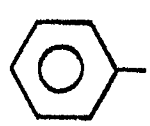 | 6 | 113 ± 8 | -14 ± 4 | 292 ± 28 | -14 ±7 |
| 2 | 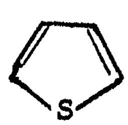 | 4 | 104 ± 8 | -12 ± 5 | 305 ± 24 | -20 ±6 |

Values are mean ± s.e. mean
N is number of animals per group.

The maximum changes in blood pressure occured between 18 and 23 mins after the administration of compound 1 , 25 to 35 mins after administration of compound 2 .

(iii) Sedative activity

m-amino Clonidine was administered orally to 6 neonatal mice at 1 mg/kg and at 10 mg/kg which were observed for 2 hours in comparison with control mice. Treated mice showed marked sedation.

(iv) Toxicity

No toxic effects were observed at the treatment doses.

(v) Piglets

2-4 Day old piglets are dosed with the compound orally 45 min prior to oral challenge with 25 ml of culture filtrate prepared from an enteropathogenic strain of *E. coli*. Control animals receive drug vehicle 45 min prior to challenge with a similar volume of material. Animals are observed for diarrhoea over a 7 hour period and the severity of scour scored on a 0-3 basis for each animal at hourly intervals. The percentage inhibition in treated animals is determined as:

$$100 - \left[\frac{\text{Mean score of scour in treated animals}}{\text{Mean score of scour in control animals}} \times 100\right]$$

Results obtained are given in Table 3.

TABLE 3

THE EFFECT OF THE HYDROCHLORIDE SALT OF THE COMPOUND OF EXAMPLE 4

ON PIGLET DIARRHOEA INDUCED BY ENTEROTOXIN ADMINISTRATION (GROUPS of 2 PIGLETS)

| DOSE p.o. (mg/kg) | DURATION OF TEST (h) | MEAN SCOUR SCORE ± SEM | o/o INHIBITION OF SCOUR | SIGNIFICANCE (P) |
|---|---|---|---|---|
| 0 | 6 | 2.7 ± 0.1 | 0 | |
| 1 | 6 | 0.4 ± 0.4 | 85 | 0.01 |
| 0.2 | 6 | 1.6 ± 0.1 | 41 | |
| 0.1 | 6 | 2.3 ± 0.5 | 15 | |
| 0 | 12 | 2.1 ± 0.2 | 0 | |
| 1 | 12 | 1.1 ± 0.2 | 48 | 0.05 |
| 0.2 | 12 | 1.2 ± 0.2 | 43 | 0.05 |
| 0.1 | 12 | 1.9 ± 0.2 | 10 | |

(vi) Rat Jejunal Perfusion

Jejunal absorption or secretion of fluid was measured in anaesthetised rats by perfusion of an isotonic solution containing $^{14}C$ - polyethylene glycol essentially as described by Klipstein, Lee and Engert (Infect. Immun. 1976, 14, 1004-1010). The effect of ST toxin alone or in the presence of drug was established by incorporating these substances in the perfusion solution averaging $^{14}C$ counts over 2 hours perfusion in six rats and calculating absorption per g of perfused jejunum.

| Perfusate | Absorption ul $min^{-1}$ $g^{-1}$ | No. of rats | $P^{(a)}$ (students t) |
|---|---|---|---|
| Control (no toxin) | + 1.3 | 20 | 0.001 |
| Toxin (60 mouse units/ml) | - 6.9 | 24 | - |
| Toxin + Compound of Example 4* 80 ug/ml | - 2.5 | 6 | 0.05 |

* as the hydrochloride salt

(a) Absorption compared to absorption by jejunum perfused with toxin alone.

0070084

SET A

CLAIMS

1. A compound of formula (I):

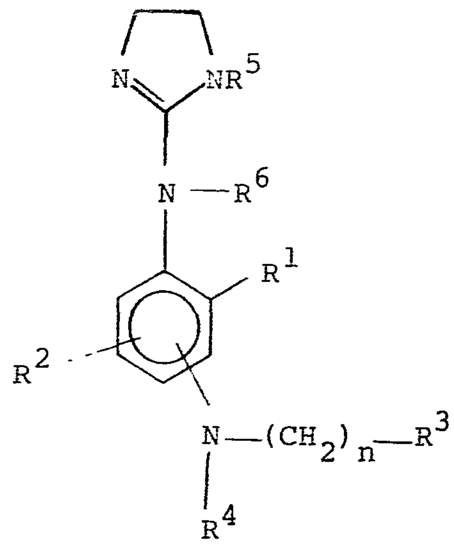

(I)

wherein $R^1$ is halogen, alkyl or alkoxy;

$R^2$ is hydrogen, halogen, alkyl or alkoxy;

$R^3$ is an optionally substituted, saturated, unsaturated or aromatic, carbo- or hetero-, mono- or bi-cyclic group;

$R^4$ is hydrogen or alkyl,

$-\underset{R^4}{\underset{|}{N}}-(CH_2)_n-R^3$ is located meta or para to the imidazolinylamino group;

one of $R^5$ and $R^6$ is hydrogen and the other is hydrogen, alkyl or acyl;

and N is 0, 1, 2 or 3,

or an acid addition salt thereof.

2. A compound as claimed in claim 1 wherein $R^3$ is selected from optionally substituted phenyl, naphthalenyl, quinolinyl, isoquinolinyl and indolyl and partially or fully saturated analogues thereof.

3. A compound as claimed in claim 1 or claim 2 wherein any substituents on the group $R^3$ are selected from halogen, alkyl, alkoxy, amino, mono- or di-alkylamino, mono- or diacylamino, nitro, hydroxy, hydroxyalkyl, alkoxyalkyl, mercapto, mercaptoalkyl, alkylthio, alkylthioalkyl, sulphonyl, sulphonamido, cyano and $-\underset{\underset{O}{\parallel}}{C}-X$ wherein X is hydroxy or an ester or amide thereof.

4. A compound as claimed in any one of claims 1 to 3 and selected from:
2-(4-benzylamino-2,6-dichlorophenylimino)-imidazolidine;
2-[4-(4-chlorobenzylamino)-2,6-dimethylphenyl]-imidazolidine;
2-[2,6-dichloro-4-(2-thenylamino)phenylimino]-imidazolidine;
2-[2,6-dichloro-4-(3,4-methylenedioxybenzylamino)-phenylimino]imidazolidine;
2-[4-(4-chlorobenzylamino)-2,6-dichlorophenylimino]imidazolidine;
2-[4-(4-carbomethoxybenzylamino)-2,6-dichlorophenylimino]imidazolidine;
2-[4-(4-carboxybenzylamino)-2,6-dichlorophenylimino]imidazolidine;
2-[2,6-dichloro-4-(4-fluorobenzylamino)phenyl-imino]imidazolidine;
2-[4-(5-bromo-2-thenylamino)-2,6-dichlorophenylimino]imidazolidine;

2-[2,6-dichloro-4-(4-methylbenzylamino)phenylimino]imidazolidine;
2-[4-(3-chlorobenzylamino)-2,6-dichlorophenylimino]imidazolidine;
2-[2,6-dichloro-4-(4-methylthiobenzylamino)phenylimino]imidazolidine;
2-[4-bromobenzylamino)-2,6-dichlorophenylimino]-imidazolidine;
2-[2,6-dichloro-4-(3-methylbenzylamino)phenylimino]imidazolidine;
2-[2,6-dichloro-4-(4-isopropylbenzylamino)phenylimino]imidazolidine;
2-(4-benzylamino-2,6-dimethylphenylimino)-imidazolidine;
2-[2,6-dimethyl-4-(3-methoxybenzylamino)phenylimino]imidazolidine;
2-[4-(4-carbomethoxybenzylamino)-2,6-dimethylphenylimino]imidazolidine;
2-(5-benzylamino-2-chlorophenylimino)-imidazolidine;
2-[5-(4-bromobenzylamino)-2-chlorophenylimino]-imidazolidine;
2-[2,6-dichloro-4-(4-hydroxymethylbenzylamino)-phenylimino]imidazolidine;
and acid addition salts thereof.

5. A process for producing a compound of formula (I) as defined in claim 1, which process comprises:

(a) reacting a compound of formula (II):

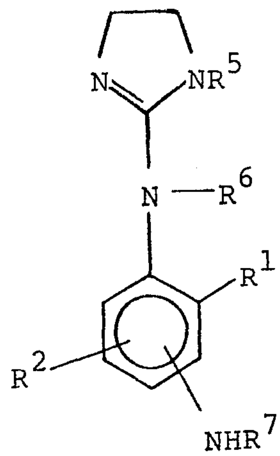

(II)

wherein $R^7$ is alkyl, $(CH_2)_nR^3$ or hydrogen;
and $R^1$, $R^2$, $R^3$, $R^5$, $R^6$ and n are as defined with respect to formula (I);

with a compound of formula (III):

$R^8 - X$

wherein $R^8$ is $(CH_2)_nR^3$ when $R^7$ is alkyl or hydrogen;
or $R^8$ is alkyl when $R^7$ is $(CH_2)_nR^3$;
and X is a reactive leaving group,

or (b) reacting a compound of formula (IV):

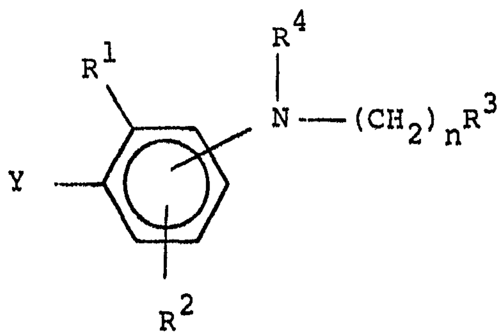

(IV)

wherein $R^1$, $R^2$, $R^3$, $R^4$ and n are as defined with respect to formula (I);

and Y is a group 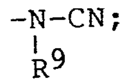

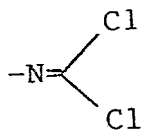 ; 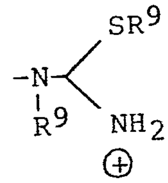 ; or

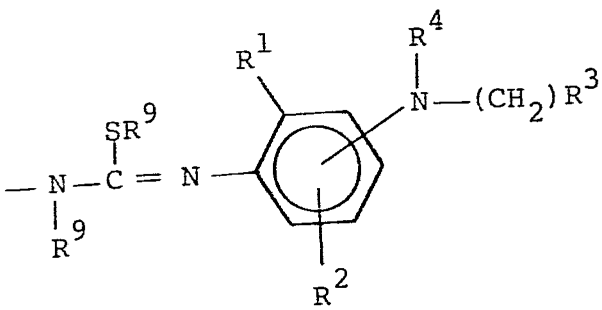

wherein each group $R^9$ is selected from hydrogen and $C_{1-4}$ alkyl;

with a compound of formula (V):

$$H_2N\text{-}CH_2CH_2\text{-}NHR^{10} \qquad (V)$$

wherein $R^{10}$ is hydrogen or $C_{1-4}$ alkyl,

or (c) reacting a compound of formula (VI):

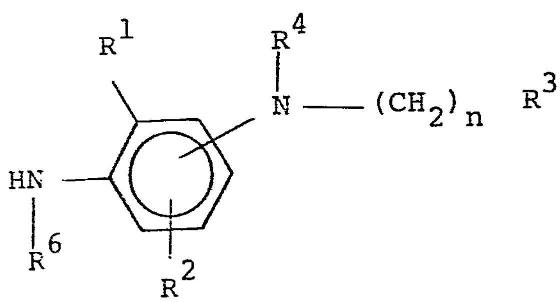

(VI)

wherein $R^1$, $R^2$, $R^3$, $R^4$, $R^6$ and n are as defined with respect to formula (I);

with a compound of formula (VII):

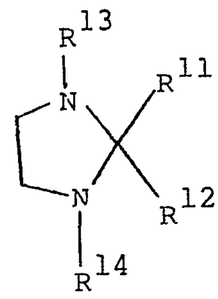

(VII)

wherein $R^{11}$ and $R^{12}$ together form an oxo group;
$R^{13}$ is hydrogen, $C_{1-4}$ alkyl or acyl;
and $R^{14}$ is hydrogen;

or $R^{12}$ and $R^{14}$ together form a bond;
$R^{11}$ is $C_{1-4}$ alkylthio or benzylthio;
and $R^{13}$ is hydrogen or $C_{1-4}$ alkyl,

or (d) reacting a compound of formula (VIII):

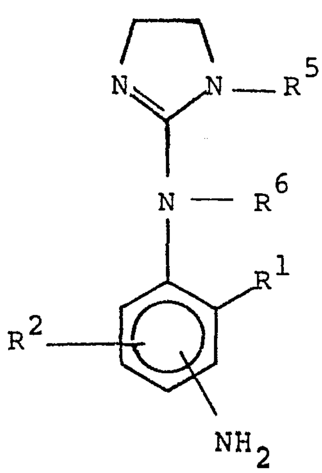

(VIII)

wherein $R^1$, $R^2$, $R^5$ and $R^6$ are as defined with respect to formula (I);

with a compound of formula (IX):

$$R^3-(CH_2)_m-\underset{\underset{O}{\|}}{C}-Z \qquad (IX)$$

wherein $R^3$ is as defined with respect to formula (I);
$m$ is 0, 1 or 2;
Z is hydrogen, halogen or $-O-\underset{\underset{O}{\|}}{C}-R^{16}$

and $R^{16}$ is hydrogen, alkyl or $-(CH_2)_m-R^3$;

and thereafter reducing the product to the requisite compound of formula (I);

and optionally (e) forming a salt of the compound of formula (I).

6. A compound of formula (IIA):

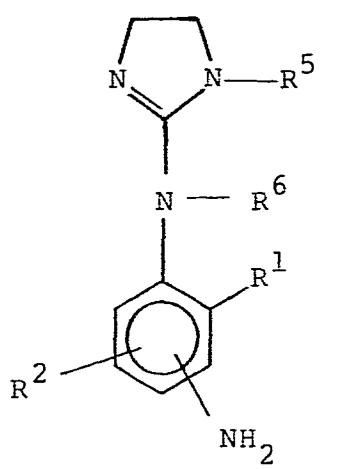

(IIA)

wherein $R^1$ is halogen, alkyl or alkoxy;
$R^2$ is hydrogen, halogen, alkyl or alkoxy;

and one of $R^5$ and $R^6$ is hydrogen and the other is hydrogen, alkyl or acyl.

7. A pharmaceutical composition comprising a compound of formula (I) as defined in claim 1 in association with a pharmaceutically acceptable carrier therefor.

8. A pharmaceutical composition comprising a compound of formula (IIA) as defined in claim 8 in association with a pharmaceutically acceptable carrier therefor.

9. A compound of formula (I) as defined in claim 1 or of formula (IIA) as defined in claim 8 for use in medicine.

10. A process for producing a pharmaceutical composition as defined in claim 7 or claim 8, which process comprises bringing into association the compound and the carrier therefor.